# EUROPEAN PATENT SPECIFICATION

(11) **EP 4 360 767 B1**
(45) Date of publication and mention of the grant of the patent: **25.03.2026**
(21) Application number: 22204493.5
(22) Date of filing: 28.10.2022
(51) Int. Cl.: B06B 1/06

(54) **AN ULTRASOUND TRANSDUCER AND A METHOD FOR PRODUCING AN ULTRASOUND TRANSDUCER**
ULTRASCHALLWANDLER UND VERFAHREN ZUR HERSTELLUNG EINES ULTRASCHALLWANDLERS
TRANSDUCTEUR ULTRASONORE ET PROCÉDÉ DE PRODUCTION D'UN TRANSDUCTEUR ULTRASONORE

(43) Date of publication of application: 01.05.2024
(73) Proprietor: Imec VZW, 3001 Leuven (BE)
(72) Inventor: Cheyns, David, 2801 Heffen (BE); Jeong, Yongbin, 3001 Heverlee (BE)
(74) Representative: AWA Sweden AB

(56) References cited:
- US-A1- 2012 319 535
- US-A1- 2020 046 320
- US-A1- 2022 152 654

## Description

### Technical field

The present inventive concept relates to the field of ultrasound imaging.

More particularly, the present inventive concept relates to a method for producing an ultrasound transducer comprising an array of ultrasound transducer elements.

### Background

Large 2D arrays of ultrasound arrays have several applications in the medical market and for consumer electronics. Examples are medical imaging, gesture recognition, directed sound, fingerprint detection and mid-air haptics.

The standard structure of a micromachined ultrasound transducer (MUT) is known in the art. A small drum is made, with a suspended membrane on top of a small cavity. The dimensions of this cavity in combination with the stiffness of the membrane will determine the resonance frequency of a particular MUT. As an example, the MUT may be driven by the piezo-electric effect (pMUT). By applying an AC electric field at the resonance frequency across a piezoelectric material, a stress difference between the piezo-electric material and the membrane is generated, and this will induce a vibration and the emission of an acoustical wave. Typical frequencies are in the range of 20 kHz to 100 MHz. This translates into wavelengths ranging from 1 cm down to <100 µm for airborne waves. Applications that use beamforming to create a focal spot in emission or to image a small spot in receiving, require larger arrays of ultrasound transducers working together.

The optimal pitch between subsequent elements in the array for ideal beam forming techniques is wavelength/2. Depending on the used stack, this means that the pitch between elements will be close to the dimensions of a discrete transducer. The consequence is a high density of pMUT elements, with almost no place between the elements.

Furthermore, a small circuit might be required next to each individual pMUT, for either setting the correct phase or reading out the phase/amplitude. This circuit might be relatively complex, and there could be no room in the same plane as the pMUTs.

US 2022/0152654 A1 discloses a method for producing a flexible ultrasound transducer for an ultrasound monitoring system for examining a curved object, in which a first layer structure and a second layer structure are processed independently on different rigid substrates.

There is thus a need in the art for improved designs of ultrasound transducers that may be manufactured in large arrays. Further, ultrasound transducers that are manufactured on flat rigid substrates may not be fit for scanning curved objects. The operator has to move and press the flat transducer against the curved object to be examined, which leads to difficulties in reproduction of images etc. Thus, there is also a need in the art for ultrasound transducers that allow for improved examination of curved objects. Furthermore, large scale ultrasound transducers may be difficult to manufacture. Thus, there is a need in the art for improved manufacturing methods.

### Summary

It is an object of the present inventive concept to at least partly overcome one or more limitations of the prior art. In particular, it is an object to provide a method for producing an ultrasound transducer suitable for large scale transducers.

This object is at least partly met by the invention as defined in the independent claim.

Preferred embodiments are set out in the dependent claims.

The invention relates to a method for producing an ultrasound transducer (1) for an ultrasound system, wherein the method comprises:
1) forming a first layer structure, said forming comprising:
   (a) forming a frontplane flexible layer above a first substrate, wherein a temporary bonding is formed between the frontplane flexible layer and the first substrate;
   (b) forming an array of ultrasound transducer elements above the frontplane flexible layer; and
2) providing a second layer structure comprising at least a backplane layer;
   wherein 1) the forming of the first layer structure further comprises and/or 2) the providing of the second layer structure comprises: 1) (c) forming a first cavity defining layer above the array of ultrasound transducer elements and/or 2) (a) forming a second cavity defining layer above the backplane layer; and defining an array of cavities in the first cavity defining layer and/or in the second cavity defining layer;
3) attaching by adhesive bonding the first layer structure to the second layer structure with the array of ultrasound transducer elements between the frontplane flexible layer and the backplane layer; and
4) removing the first substrate.

The method is for producing an ultrasound transducer, being particularly suitable for producing a large area ultrasound transducer. The ultrasound transducer may be a flexible ultrasound transducer. The method is equally particularly suitable for producing a flexible ultrasound transducer. However, it should be realized that the method need not necessarily be used for producing a large area ultrasound transducer or a flexible ultrasound transducer.

The ultrasound transducer is useful for emitting an ultrasound signal to or into an object. The ultrasound transducer may be used only for emitting an ultrasound signal to or into an object and may be used in an ultrasound system comprising an ultrasound transducer emitting an ultrasound signal. The ultrasound signal may for instance be emitted (in)to the object for causing a desired interaction between the ultrasound signal and the object, e.g., for any type of ultrasound treatment of the object.

The ultrasound transducer may further be useful for receiving an ultrasound response signal based on the emitted ultrasound signal. The ultrasound transducer may thus be used in an ultrasound monitoring system. Such system may also comprise separate processing means for processing resultant echo signals or response signals obtained from the object that is examined, display means for displaying an image obtained by the ultrasound transducer and separate communication means for transferring information from the processing means to the display means.

The method comprises forming a first layer structure and providing a second layer structure. The first layer structure and the second layer structure may be formed separately from each other. This means that the process for forming the first layer structure can be optimized for the first layer structure and the process for forming the second layer structure can be optimized for the second layer structure. This allows for optimization of the processing of the first layer and second layer respectively, before an interconnection between the layers is made. The method is compatible with forming flexible as well as rigid structures.

The first layer structure may be a multi-layer structure, such as a multi-layer structure comprising metal-insulator-metal layer stack. Such metal-insulator-metal layer stack may in some embodiments be used in providing an ultrasound transducer function based on the piezo-electric effect, the insulator being a piezoelectric material.

During the forming of the first layer structure a temperature lower than 350 °C, for example a temperature in the range between 200°C and 300°C, may be used.

The frontplane flexible layer may form a membrane which may be configured to vibrate when the ultrasound transducer is used for generating an ultrasound signal to be emitted by the ultrasound transducer. The flexible layer may be considered a "frontplane" layer in that the layer is positioned at a side of the ultrasound transducer at which ultrasound is emitted.

The array of ultrasound transducer elements is arranged in the first layer structure. The array may be a two-dimensional array. The surface area of the array may be a small surface area, such as from 1 cm² or 10 cm². The surface area of the array may be a larger area, such as at least 100 cm², such as at least 400 cm².

The array of ultrasound transducer elements is configured for generating ultrasonic energy (ultrasound signals) that propagates in a main direction, i.e., along a transducer axis Z.

The ultrasound transducer may be in the form of a sheet. The sheet may have a first outer surface and a second outer surface, oppositely arranged of each other, i.e., having normal vectors pointing in two different and parallel directions. The outer layers of the multi-layered structure may form the first and second outer sheets, respectively. The surface area of the sheet may be a small surface area, such as from 1 cm² or 10 cm². The surface area of the sheet, such as the surface area of the first or second outer surface may be a larger area, such as at least 100 cm², such as at least 400 cm².

The position of a layer or layer structure may in the present disclosure be defined as being "axially above" or "axially below" another layer or layer structure. The terms "axially" refers to the transducer axis Z, and a layer or layer structure being arranged axially above another layer or layer structure is thus arranged at a position that is further along in the positive Z direction. Hence, if the transducer axis Z points vertically upwards, then "axially above" corresponds to "vertically above" and "axially below" corresponds to "vertically below".

The resonance frequency of an ultrasound transducer element may be in the range of 20 kHz to 100 MHz. This translates into wavelengths ranging from 1 cm down to <100 µm.

The second layer structure may be a multi-layer structure. The second layer structure may alternatively comprise only the backplane layer. The term "providing the second layer structure" may thus only involve forming of the backplane layer or making the second layer structure comprising only the backplane layer available to the process of attaching the first layer structure to the second layer structure, such as bringing the second layer structure to the process of attaching the first layer structure to the second layer structure. According to other embodiments, the term "providing the second layer structure" may involve forming of a multi-layer structure.

The backplane layer may comprise a suitable substrate on which components for ultrasound transducer may be formed. The backplane layer may only function as a substrate carrying these components and may thus be formed from many different materials. In embodiments, the backplane layer may be used as a substrate on which control circuits and/or other circuitry is formed. The backplane layer may thus comprise thin-film semiconductive materials, such as IGZO (Indium Gallium Zinc Oxide) and/or LTPS (Low Temperature Poly Silicon). The backplane layer may be a flexible thin layer. The backplane layer may be a glass layer.

The backplane layer may be considered a "backplane" layer in that the layer is positioned at an opposite side to a side of the ultrasound transducer at which ultrasound is emitted.

The backplane layer may be a flexible layer or a rigid layer. When the backplane layer is a flexible layer, the entire ultrasound transducer may be flexible.

The first cavity defining layer and/or the second cavity defining layer may comprise a photoresist material. This is suitable to allow cavities to be accurately defined in the first cavity defining layer and/or the second cavity defining layer using photolithography. The cavity defining layer may be formed during step 1 (c), where the cavity defining layer may be formed above the array of ultrasound transducer elements. The cavity defining layer may alternatively be formed during step 2 (a), where the cavity defining layer is formed above the backplane layer. The cavity defining layer may in combination be formed in two separate steps, 1 (c) and 2 (a), such that a first cavity defining layer is formed in step 1 (c) and a second cavity defining layer is formed in step 2 (a), wherein the first cavity defining layer may then be attached to the second cavity defining layer for forming a combined cavity defining layer, when attaching the first layer structure to the second layer structure in step 3). In other words, the cavity defining layer may be formed as step 1 (c) above the array of ultrasound transducer elements and as step 2 (a) above the backplane layer.

If the cavity defining layer is formed only by forming the first cavity defining layer, the array of cavities will be defined only in the first cavity defining layer. If the cavity defining layer is formed only by forming the second cavity defining layer, the array of cavities will be defined only in the second cavity defining layer. If the cavity defining layer is formed by both forming the first cavity defining layer and forming the second cavity defining layer, the array of cavities may be defined only in the first cavity defining layer or only in the second cavity defining layer or in both of the first cavity defining layer and the second cavity defining layer.

The cavity defining layer may be used for bonding, in particular adhesive bonding, of the first layer structure comprising the frontplane layer and the second layer structure comprising the backplane layer. Further, the cavity defining layer may be used to define the cavities of the ultrasound transducer. The definition of the cavities may be optimized by the dimensions of the cavity defining layer. The method provided allows for micrometer resolution cavities. The cavity defining layer may have a thickness of 0.1-100 µm. The dimensions of the cavity may determine the resonance frequency of the ultrasound transducer element. The first layer structure and the second layer structure are attached to each other by adhesive bonding. The adhesive bonding may be formed by the cavity defining layer and/or by any additional layer. The first layer structure is attached to the second layer structure such that the ultrasound transducer elements are located between the frontplane flexible layer and the backplane layer. In other words, after the attachment the ultrasound transducer may have the multi-layer structure in the following order along a positive direction of the transducer axis Z: backplane layer, cavity defining layer, array of ultrasound transducer elements, frontplane layer.

By having this order of the layers, it allows for further layers to be formed on top of the frontplane layer after having removed the first substrate. This is advantageous since additional layers may then minimally affect output power or sensitivity of the array of ultrasound transducer elements.

It is a further advantage that such additional layers (for example passivation layers, acoustic impedance layers) move the neutral axis away from the piezoelectric layer of the ultrasound transducer elements.

Moreover, the array of ultrasound transducer elements are protected by the frontplane layer from environmental effects such as touching, charging and scratching and from the effect of subsequent process steps.

After the attachment of the first layer structure to the second layer structure, the first substrate is removed. By removing the first substrate at the last step, after attachment, the method allows the user to avoid the need for handling large area flexible structures. The removal of the first substrate may be made by etching the substrate. Alternatively, the removal may be made by grinding off the substrate.

The ultrasound transducer structure may be flexible or it may be rigid. The ultrasound transducer may be adapted for examination of a curved object. The curved objects suitable may be objects having a curvature with a bend radius of less than 20 cm, such as less than 10 cm. Forming the ultrasound transducer structure from flexible layers makes it possible for a flexible ultrasound transducer to bend and conform to such a curved object and an outer layer of the structure may thus form a continuous contact with the curved object.

The design of the ultrasound transducer also makes it possible to manufacture an ultrasound transducer having a large area, such as having an area that is at least 100 cm2, such as at least 400 cm2. Since the whole ultrasound transducer may be flexible, it may allow for conforming around curved objects during ultrasound imaging. A flexible ultrasound transducer of the present disclosure may thus be used for medical imaging, gesture recognition on curved substrates and non-destructive inspections around curved substrates. The curved objects or substrates that can be examined may thus be parts of the human body, such as an arm or a leg, but also other objects such as door handles and the dashboard of a car.

The flexible ultrasound transducer may be used on an object that is moving, such as on a moving human body part. Thus, the flexible ultrasound transducer may be glued or fastened onto a human body.

When processing the first layer structure and the second layer structure independently from each other, manufacturing constraints may be avoided. Hence, any temperature used for processing of the first layer structure is not limited by the thermal properties of the second layer structure (such as for example the highest temperature that the layers of the second layer structure can sustain), and vice versa.

The method further allows for the ultrasound transducer to be thin. For instance, the method may produce an ultrasound transducer having a thickness of 2-3 µm.

According to one embodiment the ultrasound transducer elements may comprise piezo-electric elements.

The piezo-electric elements may comprise scandium doped aluminum nitride (AIN). Scandium doped AIN may be suitable since piezo-electric properties can be achieved while keeping the substrate temperature during deposition in the range of 100-350°C. This range may be used while forming the ultrasound transducer elements.

Photolithography may be used while forming the piezo-electric elements.

A single ultrasound transducer element may be a piezoelectric micromachined ultrasound transducer (pMUT). A pMUT may comprise a flexible membrane on top of a small cavity. The dimensions of the cavity in combination with the stiffness of the membrane may determine the resonance frequency of the pMUT. As an example, the pMUT may be driven by the piezo-electric effect, i.e., a stress difference between a piezo-electric material and the membrane may be generated by applying an AC electric field at the resonance frequency across a piezoelectric material.

As an alternative, a single ultrasound transducer element may be a capacitive MUT (cMUT), in which the actuation is performed by an air gap in the MUT and electrodes at each side of the air gap.

According to one embodiment an ultrasound transducer element in the array may be defined by one of said piezo-electric elements and each cavity of said array of cavities is associated with a respective piezo-electric element and the piezo-electric element is arranged between the cavity and the frontplane flexible layer.

By this arrangement, the piezo-electric element is protected from any outside environmental effects which may be detrimental for the functioning.

According to one embodiment method step 2) the providing of the second layer structure may further comprise: forming the backplane layer above a second substrate.

The second substrate may be a rigid substrate or a flexible substrate. The second substrate may be formed by a glass plate. This may be useful in order to enable use of large size substrates to allow producing of large size ultrasound transducers. The second substrate may thus be adapted to handle large scale ultrasound transducers. However, it should be realized that the second substrate may be formed by other materials, such as the second substrate being formed by a semiconductor substrate or being formed by a thin-film layer.

The second substrate may function merely as a temporary carrier for carrying the backplane layer and possibly further layers during producing of the ultrasound transducer. However, the second substrate may alternatively function as a substrate that is part of the produced ultrasound transducer. This may especially be used when the second substrate is a rigid substrate and provides stability to the produced ultrasound transducer.

According to one embodiment the method may further comprise, after step 3), a step of removing the second substrate.

The removal of the second substrate may be done before or after the removal of the first substrate. In other words, the step of removal of the second substrate may be performed before step 4 of after step 4.

The removal may be made by etching the substrate. Alternatively, the removal may be made by grinding off the substrate.

The second substrate may thus be used for carrying the backplane layer when the first layer structure is attached to the second layer structure. This may facilitate handling of the second layer structure during the attaching of the first layer structure to the second layer structure. This may be particularly useful if the backplane layer is a flexible layer.

According to one embodiment the method step 2) the providing of the second layer structure may further comprise forming an acoustic backing layer above the second substrate for reducing the acoustic transmission directed away from said object during operation.

An acoustic backing layer may thus be added to the system in order to manage the wave shot that is directed away from an object that is examined. For instance, the wave shot that is directed away from an object that is examined may be reduced. An acoustic backing layer thus reduces the problems coming from acoustic emission in the wrong direction.

Furthermore, the acoustic backing layer may be an acoustic damping layer or an acoustic reflection layer in the form of a Bragg stack. The Bragg stack may comprise multiple layers of alternating high and low acoustic impedance materials.

Consequently, the acoustic backing layer may have an absorptive nature, in which the acoustic wave is substantially damped or absorbed, or a reflective nature, in which the acoustic wave is reflected.

An acoustic damping layer may be obtained by using a material in which the emitted ultrasound loses its power.

An acoustic reflection layer may be obtained by using an acoustic Bragg stack (Bragg reflector), comprising multiple layers of alternating high and low acoustic impedance materials. This may aid in reflecting the ultrasound wave. The Bragg stack may be designed using quarter wavelength rules. Thus, each of the multiple layers of the Bragg stack may have an optical thickness corresponding to one quarter of the wavelength for which the Bragg stack is designed.

According to one embodiment the method step 2) the providing of the second layer structure may further comprise: forming an array of control circuits above the backplane layer.

The array of control circuits may comprise an array of thin film transistors (TFT).

According to one embodiment the ultrasound transducer elements in the array of said first layer structure may be electrically connected with individual control circuits of the array of said second layer structure, wherein the array of control circuits is configured for operating the array of ultrasound transducer elements in said first layer structure.

Thus, an individual control circuit may be configured for controlling a single ultrasound transducer element.

As an alternative, individual control circuits in the array of control circuits may be arranged for controlling more than one ultrasound transducer element, such as at least two, such as at least four, ultrasound transducer elements. As an example, the individual control circuits may be arranged for controlling a 2x2 subarray of ultrasound transducer elements.

A TFT may comprise a semiconductor, such as IGZO (Indium Gallium Zinc Oxide) and/or LTPS (Low Temperature Poly Silicon), as well as a dielectric layer and metallic contacts. These may be deposited on a backplane layer formed on a substrate, such as polymer on a glass wafer.

There may be a single TFT associated with a single ultrasound transducer element. Thus, the ultrasound transducer may comprise a TFT backplane and a separate MUT frontplane, which are attached by the attaching of the first layer structure to the second layer structure.

The control circuits may thus be processed directly on a backplane layer, such as onto a flexible polymer layer.

It may be advantageous to have the array of ultrasound transducers and the array of control circuits in different layers, such that a control circuit is not vibrating together with the ultrasound transducer elements. Therefore, the array of ultrasound transducer elements may be provided in a frontplane whereas the array of control circuits may be provided in a backplane of the ultrasound transducer. Thus, the array of ultrasound transducer elements may be provided in the first layer structure whereas the array of control circuits may be provided in the second layer structure of the ultrasound transducer. This is further advantageous in that it allows for separate processing of frontplanes and backplanes, i.e., different layers or layer structures of the multi-layered structure may be separately produced and constraints in producing the frontplane does not affect producing of the backplane, and vice versa.

Further, since the array of ultrasound transducer elements and the array of control circuits are arranged in separate planes, it allows for using a high density of ultrasound transducer elements, such as piezoelectric micromachined ultrasound transducer elements (pMUT elements), in the array together with complex control circuits in an array below the ultrasound transducer elements.

According to one embodiment the method may further comprise the step 5) forming electrical connections between the array of ultrasound transducer elements and the array of control circuits.

The electrical connections may be made as a single connection between one control circuit and one ultrasound transducer element or it may be made in parallel, where one control circuit is connected to several ultrasound transducer elements.

According to one embodiment the step 5) forming electrical connections may comprise forming a first via through the first layer structure to a control circuit of the array of control circuits and forming a second via through the frontplane flexible layer to an ultrasound transducer element of the array of ultrasound transducer elements, and forming a metal connection between the control circuit and the ultrasound transducer element through the first via, further to the second via and through the second via.

According to one embodiment the step 5) forming electrical connections may comprise forming a via through the first layer structure including through an ultrasound transducer element of the array of ultrasound transducer elements to a control circuit of the array of control circuits and forming a metal connection between the control circuit and the ultrasound transducer element through the via.

The via may have a first, large cross-sectional size extending only through the frontplane flexible layer to an ultrasound transducer element and a second, small cross-sectional size extending further through the first layer structure to the control circuit.

The forming of the electrical connections by forming the metal connection between the control circuit and the ultrasound transducer element through the via may be advantageous in ensuring efficient use of area for forming the ultrasound transducer. However, for ease of production of the ultrasound transducer, the forming the metal connection between the control circuit and the ultrasound transducer element through the first via, further to the second via and through the second via as defined above may be preferable.

According to one embodiment the method may further comprise arranging an integrated circuit structure above or below the backplane layer and connecting the integrated circuit structure to the array of control circuits.

The function of the integrated circuit structure can be multiple, such as providing signals for excitation of the ultrasound transducer elements, performing the readout of the signals of the ultrasound transducer elements. Thus, the integrated circuit structure may be configured for processing the resultant echo signals obtained from the object that is examined. Moreover, the integrated circuit structure may also provide for wireless communication with other units, such as a display system, of an ultrasound monitoring system.

The integrated circuit structure may comprise any standard or custom device, such as Complementary Metal Oxide Semiconductor (CMOS) devices, to support specific functionalities, e.g., wireless ASIC or Digital Signal Processors (DSP).

The integrated circuit structure may comprise a plurality of Application Specific Integrated Circuits (ASIC). As an example, at least one ASIC may support a plurality of individual control circuits.

The control circuits may thus be driven by a plurality of dedicated ASICs, and a single ASIC may thus be configured for supporting or driving a plurality of individual control circuits.

An ASIC may be specially built for performing a specific function or a specific task and may be configured for exciting the ultrasound transducer elements but also for reading output signals from the array of ultrasound transducer elements. The plurality of ASICs may also be configured for wireless communication with other parts of an ultrasound monitoring system.

As an example, the plurality of ASICs may be mounted as discrete elements axially below the backplane layer.

Consequently, the ASICs may be mounted directly onto the backplane layer, using e.g., chip-on-flex techniques.

However, as an alternative, the ASICs may be incorporated in one of the layers of the second layer structure.

According to one embodiment the method may further comprise, after 4), adding an additional layer above the frontplane flexible layer.

The additional layer may be a passivation layer or an acoustic matching layer.

By the method of the invention, any additional layer may further be formed on top of the frontplane layer and the array of ultrasound transducer elements will not be arranged between the additional layer and the frontplane layer. This is an advantage of the method for producing the ultrasound transducer compared to arranging any additional layer on the array of ultrasound transducers. The arranging of the additional layer above the frontplane layer will move a neutral axis around which vibrations in the frontplane layer occurs away from the ultrasound transducer elements. Thus, the neutral axis may be maintained within the frontplane layer instead of moving into the layers forming the ultrasound transducer elements (if these are arranged above the frontplane layer), such that decrease of output power or sensitivity of the ultrasound transducer due to the forming of the additional layer above the frontplane layer may be avoided.

According to one embodiment the method the step 1) the forming of the first layer structure and the step 2) the providing of the second layer structure are processed separately from each other.

This allows for separate processing environments. Thus, the process for forming the first layer structure can be optimized for the first layer structure and the process for forming the second layer structure can be optimized for forming the second layer structure. This allows for optimization of the processing of the first layer and second layer respectively, before an interconnection between the layers is made.

According to a second aspect not encompassed by the wording of the claims, an ultrasound transducer for an ultrasound system is provided, wherein said ultrasound transducer comprises a multi-layer structure comprising a first layer structure and a second layer structure,
wherein said first layer structure comprises
an array of ultrasound transducer elements arranged in the first layer structure and configured for generating ultrasonic energy propagating along a positive direction of a main transducer axis Z; and
a frontplane flexible layer arranged at a position that is further along the positive direction of the main transducer axis Z than said array of ultrasound transducer elements;
wherein said second layer structure comprises
a backplane layer, and
wherein the multilayer structure further comprises a cavity defining layer between the backplane layer and the array of ultrasound transducer elements, wherein an array of cavities is defined in the cavity defining layer; and
wherein the first layer structure is adhesively bonded to the second layer structure.

This aspect may generally present the same or corresponding advantages as the first aspect.

The array of ultrasound transducer elements may comprise piezo-electric elements. Thus, a single ultrasound transducer element may be a piezoelectric micromachined ultrasound transducer element (pMUT element). A pMUT element may comprise a flexible membrane on top of a small cavity.

The frontplane flexible layer may form the membrane which may be configured to vibrate when the ultrasound transducer is used in order for ultrasound signal to be emitted by the ultrasound transducer.

It is an advantage that the array of ultrasound transducer elements are embedded between the frontplane flexible layer and the backplane layer. This protects the ultrasound transducer elements from environmental impacts. It further allows the multi-layered structure to have further layers above the frontplane flexible layer.

The backplane layer may be a flexible layer or a rigid layer. The backplane layer may comprise a suitable substrate on which components for ultrasound transducer may be formed. The backplane layer may only function as a substrate carrying these components and may thus be formed from many different materials. In embodiments, the backplane layer may be used as a substrate on which control circuits and/or other circuitry is formed. The backplane layer may thus comprise thin-film semiconductive materials, such as IGZO (Indium Gallium Zinc Oxide) and/or LTPS (Low Temperature Poly Silicon). The backplane layer may further be arranged above a second substrate.

The cavity defining layer may comprise a photoresist material. This is suitable to allow cavities to be accurately defined in the cavity defining layer using photolithography. The cavity defining layer defines the cavities under the array of ultrasound transducer elements. The cavity defining layer may be arranged such that each ultrasound transducer element may have a single cavity. The cavity defining layer may be a part of the first layer structure or the second layer structure or a part of both of the first layer structure and the second layer structure.

The second layer structure may further comprise an acoustic backing layer being arranged above the second substrate. The acoustic backing layer may be configured for reducing the acoustic transmission directed away from the object during operation. The acoustic backing layer may be an acoustic damping layer or an acoustic reflection layer in the form of a Bragg stack. The Bragg stack may comprise multiple layers of alternating high and low acoustic impedance materials.

Consequently, the acoustic backing layer may have an absorptive nature, in which the acoustic wave is substantially damped or absorbed, or a reflective nature, in which the acoustic wave is reflected.

The multi-layer structure may further comprise control circuits. The control circuits may be arranged in the second layered structure. The control circuits may be arranged in the second layered structure at a position that is further along a positive direction of the main transducer axis Z compared to the backplane layer. The control circuits may be configured for operating the array of ultrasound transducer elements. Each ultrasound transducer element may be operated by a single control circuit or a control circuit may operate multiple ultrasound transducer elements in parallel.

The array of control circuits may comprise an array of thin film transistors (TFT). A TFT may comprise a semiconductor, such as IGZO (Indium Gallium Zinc Oxide) and/or LTPS (Low Temperature Poly Silicon), as well as a dielectric layer and metallic contacts. These may be deposited on a backplane layer forming a substrate, such as polymer on a glass wafer.

There may be a single TFT associated with a single ultrasound transducer element. Thus, the ultrasound transducer may comprise a TFT backplane and a separately produced pMUT frontplane.

The multi-layered structure may further comprise electrical connections between the array of ultrasound transducer elements and the array of control circuits. The electrical connections may be formed by a first via through the first layer structure to a control circuit of the array of control circuits and a second via through the frontplane flexible layer to an ultrasound transducer element of the array of ultrasound transducer elements, such that a metal connection is formed between the control circuit and the ultrasound transducer element through the first via, further to the second via and through the via second via. Alternatively, the electrical connections may be formed by a via through the first layer structure including through an ultrasound transducer element of the array of ultrasound transducer elements to a control circuit of the array of control circuits and forming a metal connection between the control circuit and the ultrasound transducer element through the via. The via may have a first, large cross-sectional size extending only through the frontplane flexible layer to an ultrasound transducer element and a second, small cross-sectional size extending further through the first layer structure to the control circuit.

Even further, the multi-layered structure may comprise an integrated circuit structure above or below the backplane layer. The integrated circuit structure may be electrically connected to the array of control circuits.

The integrated circuit structure may comprise any standard or custom device, such as Complementary Metal Oxide Semiconductor (CMOS) devices, to support specific functionalities, e.g. wireless ASIC or Digital Signal Processors (DSP).

The integrated circuit structure may comprise a plurality of Application Specific Integrated Circuits (ASIC). As an example, at least one ASIC may support a plurality of individual control circuits.

The control circuits may thus be driven by a plurality of dedicated ASICs, and a single ASIC may thus be configured for supporting or driving a plurality of individual control circuits.

An ASIC may be specially built for performing a specific function or a specific task and may be configured for exciting the ultrasound transducer elements but also for reading output signals from the array of ultrasound transducer elements. The plurality of ASICs may also be configured for wireless communication with other parts of an ultrasound monitoring system.

As an example, the plurality of ASICs may be mounted as discrete elements axially below the backplane layer.

Consequently, the ASICs may be mounted directly onto the backplane layer, using e.g., chip-on-flex techniques.

However, as an alternative, the ASICs may be incorporated in one of the layers of the second layer structure.

The multi-layer structure may further comprise an additional layer above the frontplane layer. The additional layer may be a passivation layer or an acoustic matching layer.

It is an advantage compared to structures having a different layer structure that the ultrasound transducer elements are embedded below the frontplane layer in the present invention. This allows for easier addition of extra layers without affecting the ultrasound transducer elements.

The multi-layer structure may be a flexible structure or a rigid structure. A flexible structure may be configured such that the ultrasound transducer can form a continuous contact with a curved object during operation.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a schematic drawing of the method for producing an ultrasound transducer for an ultrasound system.
Fig. 2 is a schematic drawing of the method for producing an ultrasound transducer for an ultrasound system.
Fig. 3a-3g is a side view of the ultrasound transducer system according to an embodiment of the method for producing the same.
Fig. 4a-4c is a side view of the ultrasound transducer system according to another embodiment of the method for producing the same.
Fig. 5a-5d is a side view of the ultrasound transducer system according to yet another embodiment of the method for producing the same.

### Detailed description

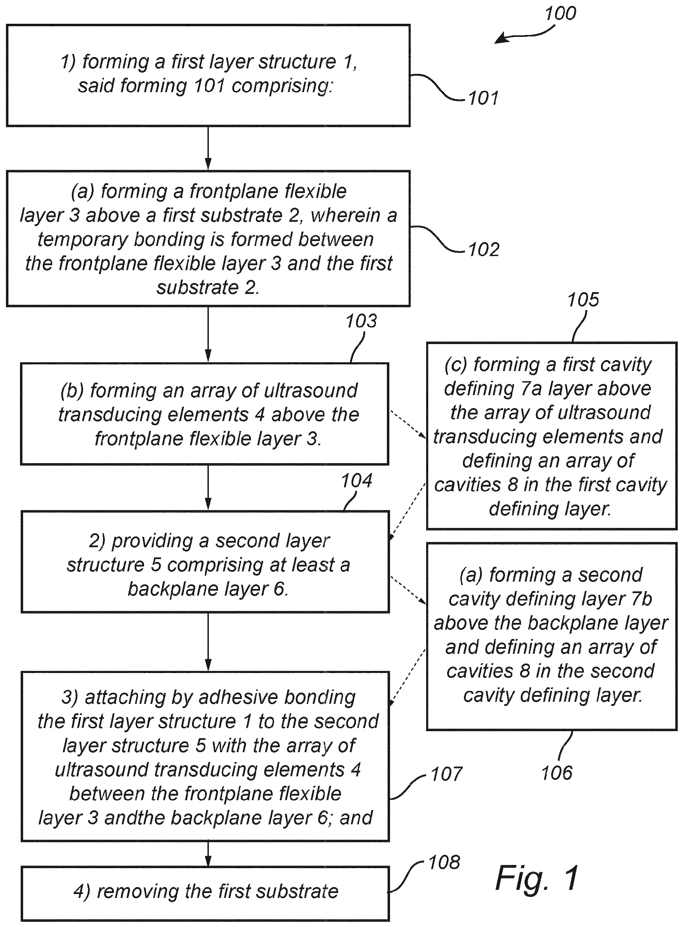

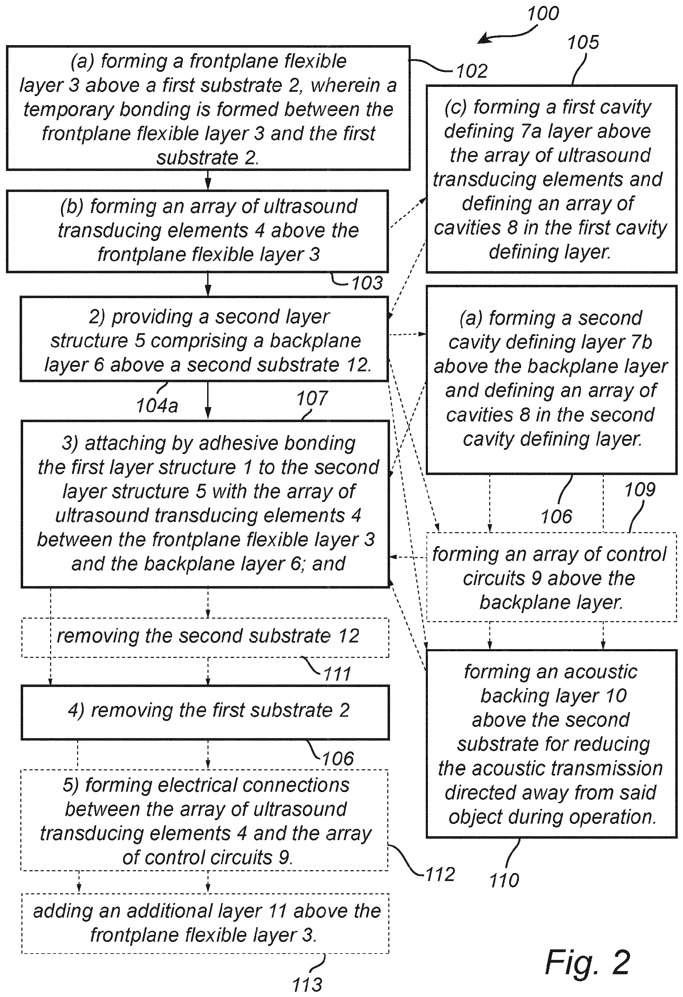

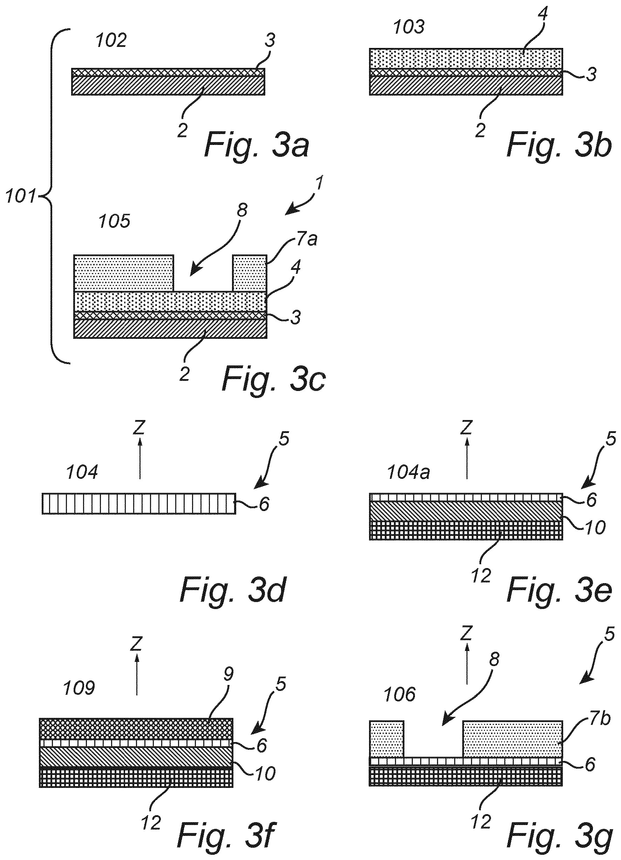

Referring now to Figs 1 and 2, to a method 100 for producing an ultrasound transducer 200 for an ultrasound system will be described. The method 100 comprises the below general method steps 1) - 4), illustrated schematically in Figs. 1 and 2. Each step will be more thoroughly discussed below in relation to Figs. 3 to 5. The method 100 comprises the steps:
1) forming 101 a first layer structure 1, said forming 101 comprising:
   (a) forming 102 a frontplane flexible layer 3 above a first substrate 2, wherein a temporary bonding is formed between the frontplane flexible layer 3 and the first substrate 2;
   (b) forming 103 an array of ultrasound transducer elements 4 above the frontplane flexible layer 3; and
2) providing 104 a second layer structure 5 comprising at least a backplane layer 6;
   wherein 1) the forming 101 of a first layer structure further comprises and/or 2) the providing 104 of a second layer structure comprises: 1) (c) forming 105 a first cavity defining 7a layer above the array of ultrasound transducer elements and/or 2) (a) forming 106 a second cavity defining layer 7b above the backplane layer; and defining an array of cavities 8 in the first cavity defining layer and/or in the second cavity defining layer;
3) attaching 107 by adhesive bonding the first layer structure 1 to the second layer structure 5 with the array of ultrasound transducer elements 4 between the frontplane flexible layer 3 and the backplane layer 6; and
4) removing 108 the first substrate 2.

Thus, in one embodiment the method may comprise the steps:
1) forming 101 a first layer structure 1, said forming 101 comprising:
   (a) forming 102 a frontplane flexible layer 3 above a first substrate 2, wherein a temporary bonding is formed between the frontplane flexible layer 3 and the first substrate 2;
   (b) forming 103 an array of ultrasound transducer elements 4 above the frontplane flexible layer 3;
   (c) forming 105 a first cavity defining 7a layer above the array of ultrasound transducer elements and defining an array of cavities 8 in the first cavity defining layer; and
2) providing 104 a second layer structure 5 comprising at least a backplane layer 6;
3) attaching 107 by adhesive bonding the first layer structure 1 to the second layer structure 5 with the array of ultrasound transducer elements 4 between the frontplane flexible layer 3 and the backplane layer 6; and
4) removing 108 the first substrate 2.

In a further embodiment the method may comprise the steps:
1) forming 101 a first layer structure 1, said forming 101 comprising:
   (a) forming 102 a frontplane flexible layer 3 above a first substrate 2, wherein a temporary bonding is formed between the frontplane flexible layer 3 and the first substrate 2;
   (b) forming 103 an array of ultrasound transducer elements 4 above the frontplane flexible layer 3; and
2) providing 104 a second layer structure 5 comprising at least a backplane layer 6;
   (a) forming 106 a second cavity defining layer 7b above the backplane layer and defining an array of cavities 8 in the second cavity defining layer;
3) attaching 107 by adhesive bonding the first layer structure 1 to the second layer structure 5 with the array of ultrasound transducer elements 4 between the frontplane flexible layer 3 and the backplane layer 6; and
4) removing 108 the first substrate 2.

Moreover, in an even further embodiment the method may comprise the steps:
1) forming 101 a first layer structure 1, said forming 101 comprising:
   (a) forming 102 a frontplane flexible layer 3 above a first substrate 2, wherein a temporary bonding is formed between the frontplane flexible layer 3 and the first substrate 2;
   (b) forming 103 an array of ultrasound transducer elements 4 above the frontplane flexible layer 3;
   (c) forming 105 a first cavity defining 7a layer above the array of ultrasound transducer elements and defining an array of cavities in the first cavity defining layer; and
2) providing 104 a second layer structure 5 comprising at least a backplane layer 6;
   (a) forming 106 a second cavity defining layer 7b above the backplane layer and defining an array of cavities 8 in the second cavity defining layer;
3) attaching 107 by adhesive bonding the first layer structure 1 to the second layer structure 5 with the array of ultrasound transducer elements 4 between the frontplane flexible layer 3 and the backplane layer 6; and
4) removing 108 the first substrate 2.

Fig. 2 illustrates further embodiments of the present invention. The step 2) of providing 104a a second layer structure 5 may in one embodiment further comprise forming the second layer structure 5 above a second substrate 12. The step 2) of forming 104, 104a a second layer structure 5 may further comprise forming 109 an array of control circuits 9 above the backplane layer 6. Further the step 2) of forming 104a the second layer structure 5 may comprise forming 110 an acoustic backing layer 10 above the second substrate 12.

Further, the method 100 may comprise a step 111 of removing the second substrate 12. This step may be performed before the method step 4).

Even further, the method may comprise a step 5) forming 112 electrical connections 13 between the array of ultrasound transducer elements 4 and the array of control circuits 9.

The method may also comprise a step of adding 113 an additional layer 11 above the frontplane flexible layer 3. The step 113 is performed after the removal 108 of the first substrate 2.

Figs. 3-5 shows schematically how the method 100 is made to produce an ultrasound transducer 200 for an ultrasound system. In other words, Figs. 3-5 schematically shows how the different layer systems may be arranged during the method 100.

Fig. 3a-3c discloses the step 1) of forming 101 a first layer structure. The frontplane flexible layer 3 is formed above a first substrate 2. The first substrate 2 being a rigid substrate, which may comprise a glass substrate. The frontplane flexible layer 3 may comprise a membrane material. The membrane material may be a polymer, such as poly-imide or an inorganic dielectrics such as SiO₂ or Si₃N₄. The bonding between the frontplane flexible layer 3 and the first substrate 2 is a temporary bonding, allowing for a removal of the first substrate 2 later in the method.

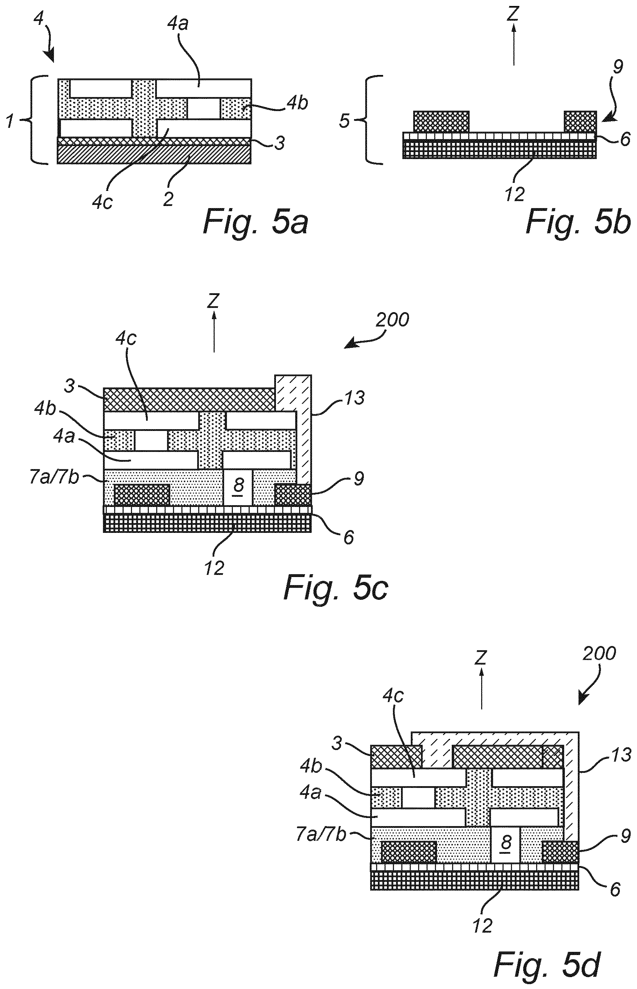

Following is the forming 103 of an array of ultrasound transducer elements 4 above the frontplane flexible layer 3. The ultrasound transducer elements 4 may comprise piezo-electric elements. Further, the first layer structure 1 may comprise a first cavity defining layer 7a, formed 105 above the array of ultrasound transducer elements 4. The first cavity defining layer 7a defines an array of cavities 8. Thus, an ultrasound transducer element in the array 4 may be defined by one of said piezo-electric elements and each of said array of cavities 8 may be associated with a respective piezo-electric element. The piezo-electric elements are more discussed in relation to Figs. 5a-5c.

Figs. 3d-3g discloses the step 2) of providing 104 a second layer structure 5 comprising at least a backplane layer 6. The backplane layer 6 may comprise a flexible thin layer. In one embodiment, shown in Fig. 3e the backplane layer 6 is further arranged 104a above a second substrate 12. The second substrate 12 may be a glass substrate. Alternatively, the backplane layer 6 may comprise a glass substrate. The second layer structure 5 may further comprise an acoustic backing layer 10 being arranged above the second substrate 12. The acoustic backing layer may be an acoustic damping layer or an acoustic reflection layer in the form of a Bragg stack. The Bragg stack may comprise multiple layers of alternating high and low acoustic impedance materials.

Even further, as disclosed in Fig. 3f the second layer structure 5 may further comprise an array of control circuits 9. The control circuits 9 may be arranged in the second layer structure 5 at a position that is further along a positive direction of the main transducer axis Z compared to the backplane layer 6. The control circuits 9 may be configured for operating the array of ultrasound transducer elements 4. Each ultrasound transducer element 4 may be operated by a single control circuit 9 or a control circuit 9 may operate multiple ultrasound transducer elements 4 in parallel. The array of control circuits 9 may comprise an array of thin film transistors (TFT). A TFT may comprise a semiconductor, such as IGZO (Indium Gallium Zinc Oxide) and/or LTPS (Low Temperature Poly Silicon), as well as a dielectric layer and metallic contacts. These may be deposited on a substrate, such as polymer on a glass wafer. There may be a single TFT associated with a single ultrasound transducer element 4.

As shown in Fig. 3g, the second layer structure 5 may further comprise a second cavity defining layer 7b. The second cavity defining layer 7b may be formed 106 on top of the backplane layer 6 as shown in Fig. 3g. Alternatively, the second cavity defining layer 7b may be further formed on top of the array of control circuits 9. The second cavity defining layer 7b defines an array of cavities 8 in the second cavity defining layer 7b.

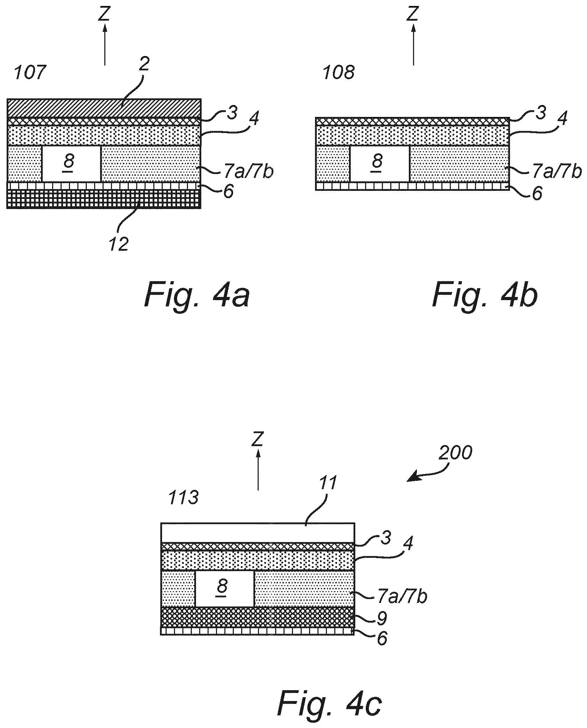

Fig. 4a schematically shows the method step 3) of attaching 107 by adhesive bonding the first layer structure 1 to the second layer structure 5. The attachment is made such that the array of ultrasound transducer elements 4 are arranged between the frontplane flexible layer 3 and the backplane layer 6.

The first layer structure 1 and the second layer structure 5 may be manufactured separately, under different environmental circumstances before they are attached together.

The cavity defining layer 7a, 7b may be arranged on the first layer structure 1, the second layer structure 5 or on both structures 1, 5.

Fig. 4b shows one embodiment of the ultrasound transducer 200 after the step 4) of removing 108 the first substrate 2 as well as after removal of the second substrate 12. The second substrate 12 may alternatively be kept on the ultrasound transducer 200. The removing 108 may be made by etching the substrate. Alternatively, the removing 108 may be made by grinding off the substrate.

Fig. 4c shows the ultrasound transducer 200 with an additional layer 11 above the frontplane flexible layer 3. Further, in Fig. 4c, the electrical connections 13 between the array of ultrasound transducer elements 4 and the array of control circuits 9 are shown. The electrical connections 13 are arranged through the cavity defining layer 7a, 7b and is used by the control circuits 9 to apply an electric field over the ultrasound transducer element 4. The electrical connections 13 are formed as a metal connection between the control circuit and the ultrasound transducer element through a via.

In Fig. 5a an embodiment of the ultrasound transducer element 4 is schematically shown. For clarity reasons, the first substrate 2 and the frontplane flexible layer 3 are shown. In this embodiment, the ultrasound transducer element 4 comprises piezo-electric elements. The piezo electric element comprises a first metal layer 4a, a piezo-electric material layer 4b and a second metal layer 4c.

In Fig. 5b an embodiment of the second layer structure 5 is disclosed. The second layer structure 5 comprises the second substrate 12, the backplane layer 6 and the array of control circuits 9 comprising an array of TFTs.

In Fig. 5c an embodiment is disclosed where the first layer structure 1 is attached by adhesive bonding to the second layer structure 5. The cavity defining layer 7a, 7b defining the cavity 8 is arranged between the ultrasound transducer elements 4 and the control circuits 9. The electrical connection 13 connects the control circuit 9 to the ultrasound transducer elements 4.

In Fig. 5d an embodiment is disclosed where the electrical connection 13 is formed by a first via through the first layer structure 1 to the control circuit of the array of control circuits 9 and by forming a second via through the frontplane flexible layer 3 to the ultrasound transducer element of the array of ultrasound transducer elements 4. A metal connection is formed between the control circuit 9 and the ultrasound transducer element 4 through the first via and further to the second via and through the second via.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A method (100) for producing an ultrasound transducer (200) for an ultrasound system, wherein the method comprises:
1) forming (101) a first layer structure (1), said forming (101) comprising:
(a) forming (102) a frontplane flexible layer (3) above a first substrate (2), wherein a temporary bonding is formed between the frontplane flexible layer (3) and the first substrate (2);
(b) forming (103) an array of ultrasound transducer elements (4) above the frontplane flexible layer (3); and
2) providing (104) a second layer structure (5) comprising at least a backplane layer (6);
wherein 1) the forming (102) of the first layer structure (1) further comprises and/or 2) the providing (104) of the second layer structure (5) comprises: 1) (c) forming (105) a first cavity defining layer (7a) above the array of ultrasound transducer elements (4) and/or 2) (a) forming (106) a second cavity defining layer (7b) above the backplane layer (6); and defining an array of cavities (8) in the first cavity defining layer (7a) and/or in the second cavity defining layer (7b);
3) attaching (107) by adhesive bonding the first layer structure (1) to the second layer structure (5) with the array of ultrasound transducer elements (4) between the frontplane flexible layer (3) and the backplane layer (6); and
4) removing (108) the first substrate (2).

2. A method (100) according to claim 1, wherein the ultrasound transducer elements (4) comprise piezo-electric elements.

3. A method (100) according to claim 2, wherein an ultrasound transducer element (4) in the array is defined by one of said piezo-electric elements and wherein each cavity of said array of cavities (8) is associated with a respective piezo-electric element and the piezo-electric element is arranged between the cavity (8) and the frontplane flexible layer (3).

4. A method (100) according to any one of the preceding claims, wherein 2) the providing (104) of the second layer structure (5) comprises: forming (104a) the backplane layer (6) above a second substrate (12).

5. A method (100) according to claim 4, wherein the method further comprises, after 3), removing (111) the second substrate (12).

6. A method (100) according to any one of claims 4 or 5, wherein 2) the providing (104) of the second layer structure (5) further comprises forming (110) an acoustic backing layer (10) above the second substrate (12) for reducing the acoustic transmission directed away from said object during operation.

7. A method (100) according to any one of the preceding claims, wherein 2) the providing (104) of the second layer structure (5) comprises: forming (109) an array of control circuits (9) above the backplane layer (6).

8. A method (100) according to claim 7, wherein the ultrasound transducer elements (4) in the array of said first layer structure (1) are electrically connected with individual control circuits (9) of the array of said second layer structure (5), wherein the array of control circuits (9) is configured for operating the array of ultrasound transducer elements (4) in said first layer structure (1).

9. A method (100) according to claim 8, wherein the method further comprises 5) forming (112) electrical connections (13) between the array of ultrasound transducer elements (4) and the array of control circuits (9).

10. A method (100) according to claim 9, wherein 5) forming electrical connections (13) comprises forming a first via through the first layer structure (1) to a control circuit of the array of control circuits (9) and forming a second via through the frontplane flexible layer (3) to an ultrasound transducer element of the array of ultrasound transducer elements (4), and forming a metal connection between the control circuit (9) and the ultrasound transducer element (4) through the first via, further to the second via and through the second via.

11. A method (100) according to claim 9, wherein 5) forming (112) electrical connections (13) comprises forming a via through the first layer structure (1) including through an ultrasound transducer element of the array of ultrasound transducer elements (4) to a control circuit of the array of control circuits (9) and forming a metal connection between the control circuit (9) and the ultrasound transducer element (4) through the via.

12. A method (100) according to any one of claims 7-11, further comprising arranging an integrated circuit structure above or below the backplane layer (6) and connecting the integrated circuit structure to the array of control circuits (9).

13. A method (100) according to any one of the preceding claims, further comprising, after 4), adding (113) an additional layer (11) above the frontplane flexible layer (3).

14. A method (100) according to any one of the preceding claims, wherein 1) the forming (101) of the first layer structure (1) and 2) the providing (104) of the second layer structure (5) are processed separately from each other.

## Patentansprüche

1. Verfahren (100) zum Herstellen eines Ultraschallwandlers (200) für ein Ultraschallsystem, wobei das Verfahren umfasst:
1) Bilden (101) einer ersten Schichtstruktur (1), wobei das Bilden (101) umfasst:
(a) Bilden (102) einer biegsamen Vorderwandplatinenschicht (3) über einem ersten Substrat (2), wobei eine vorübergehende Bindung zwischen der biegsamen Vorderwandplatinenschicht (3) und dem ersten Substrat (2) entsteht;
(b) Bilden (103) einer Anordnung von Ultraschallwandlerelementen (4) über der biegsamen Vorderwandplatinenschicht (3); und
2) Bereitstellen (104) einer zweiten Schichtstruktur (5), die mindestens eine Rückwandplatinenschicht (6) umfasst;
wobei Schritt 1) des Bildens (102) der ersten Schichtstruktur (1) ferner umfasst und/oder Schritt 2) des Bereitstellens (104) der zweiten Schichtstruktur (5) umfasst: 1) (c) Bilden (105) einer ersten Schicht (7a), die einen Hohlraum definiert, über der Anordnung von Ultraschallwandlerelementen (4) und/oder 2) (a) Bilden (106) einer zweiten Schicht (7b), die einen Hohlraum definiert, über der Rückwandplatinenschicht (6); und Definieren einer Anordnung von Hohlräumen (8) in der ersten Schicht (7a), die einen Hohlraum definiert, und/oder in der zweiten Schicht (7b), die einen Hohlraum definiert;
3) Anbringen (107) durch Klebebindung der ersten Schichtstruktur (1) an der zweiten Schichtstruktur (5), wobei sich die Anordnung von Ultraschallwandlerelementen (4) zwischen der biegsamen Vorderwandplatinenschicht (3) und der Rückwandplatinenschicht (6) befindet; und
4) Entfernen (108) des ersten Substrats (2).

2. Verfahren (100) nach Anspruch 1, wobei die Ultraschallwandlerelemente (4) piezoelektrische Elemente umfassen.

3. Verfahren (100) nach Anspruch 2, wobei ein Ultraschallwandlerelement (4) in der Anordnung durch eines der piezoelektrischen Elemente definiert ist, und wobei jeder Hohlraum der Anordnung von Hohlräumen (8) einem jeweiligen piezoelektrischen Element zugeordnet ist, und das piezoelektrische Element zwischen dem Hohlraum (8) und der biegsamen Vorderwandplatinenschicht (3) angeordnet ist.

4. Verfahren (100) nach einem der vorhergehenden Ansprüche, wobei Schritt 2) des Bereitstellens (104) der zweiten Schichtstruktur (5) umfasst: Bilden (104a) der Rückwandplatinenschicht (6) über einem zweiten Substrat (12).

5. Verfahren (100) nach Anspruch 4, wobei das Verfahren ferner nach Schritt 3) das Entfernen (111) des zweiten Substrats (12) umfasst.

6. Verfahren (100) nach einem der Ansprüche 4 oder 5, wobei Schritt 2) des Bereitstellens (104) der zweiten Schichtstruktur (5) ferner das Bilden (110) einer akustischen Trägerschicht (10) über dem zweiten Substrat (12) zum Reduzieren der Schallübertragung, die von dem Objekt weg gerichtet ist, während des Betriebs umfasst.

7. Verfahren (100) nach einem der vorhergehenden Ansprüche, wobei Schritt 2) des Bereitstellens (104) der zweiten Schichtstruktur (5) umfasst: Bilden (109) einer Anordnung von Steuerschaltungen (9) über der Rückwandplatinenschicht (6).

8. Verfahren (100) nach Anspruch 7, wobei die Ultraschallwandlerelemente (4) in der Anordnung der ersten Schichtstruktur (1) mit einzelnen Steuerschaltungen (9) der Anordnung der zweiten Schichtstruktur (5) elektrisch verbunden sind, wobei die Anordnung von Steuerschaltungen (9) dazu konfiguriert ist, die Anordnung von Ultraschallwandlerelementen (4) in der ersten Schichtstruktur (1) zu betätigen.

9. Verfahren (100) nach Anspruch 8, wobei das Verfahren ferner einen Schritt 5) des Bildens (112) von elektrischen Verbindungen (13) zwischen der Anordnung von Ultraschallwandlerelementen (4) und der Anordnung von Steuerschaltungen (9) umfasst.

10. Verfahren (100) nach Anspruch 9, wobei Schritt 5) des Bildens von elektrischen Verbindungen (13) das Bilden eines ersten Durchgangslochs durch die erste Schichtstruktur (1) hindurch bis zu einer Steuerschaltung der Anordnung von Steuerschaltungen (9) und das Bilden eines zweiten Durchgangslochs durch die biegsame Vorderwandplatinenschicht (3) hindurch bis zu einem Ultraschallwandlerelement der Anordnung von Ultraschallwandlerelementen (4), und das Bilden einer Metallverbindung zwischen der Steuerschaltung (9) und dem Ultraschallwandlerelement (4) durch das erste Durchgangsloch hindurch, weiter bis zu dem zweiten Durchgangsloch und durch das zweite Durchgangsloch hindurch umfasst.

11. Verfahren (100) nach Anspruch 9, wobei Schritt 5) des Bildens (112) von elektrischen Verbindungen (13) das Bilden eines Durchgangslochs durch die erste Schichtstruktur (1) hindurch, einschließlich durch ein Ultraschallwandlerelement der Anordnung von Ultraschallwandlerelementen (4) hindurch bis zu einer Steuerschaltung der Anordnung von Steuerschaltungen (9) und das Bilden einer Metallverbindung zwischen der Steuerschaltung (9) und dem Ultraschallwandlerelement (4) durch das Durchgangsloch hindurch umfasst.

12. Verfahren (100) nach einem der Ansprüche 7 bis 11, ferner umfassend das Anordnen einer integrierten Schaltungsstruktur über oder unter der Rückwandplatinenschicht (6) und das Verbinden der integrierten Schaltungsstruktur mit der Anordnung von Steuerschaltungen (9).

13. Verfahren (100) nach einem der vorhergehenden Ansprüche, ferner umfassend nach Schritt 4) das Hinzufügen (113) einer zusätzlichen Schicht (11) über der biegsamen Vorderwandplatinenschicht (3).

14. Verfahren (100) nach einem der vorhergehenden Ansprüche, wobei Schritt 1) des Bildens (101) der ersten Schichtstruktur (1) und Schritt 2) des Bereitstellens (104) der zweiten Schichtstruktur (5) voneinander getrennt verarbeitet werden.

## Revendications

1. Procédé (100) de fabrication d'un transducteur ultrasonore (200) pour un système ultrasonore, dans lequel le procédé comprend :
1) la formation (101) d'une première structure de couche (1), ladite formation (101) comprenant :
(a) la formation (102) d'une couche flexible frontale (3) au-dessus d'un premier substrat (2), dans lequel une liaison temporaire est établie entre la couche flexible frontale (3) et le premier substrat (2) ;
(b) la formation (103) d'un réseau d'éléments de transducteur ultrasonore (4) au-dessus de la couche flexible frontale (3) ; et
2) la réalisation (104) d'une deuxième structure de couche (5) comprenant au moins une couche de plaque arrière (6) ;
dans lequel 1) la formation (102) de la première structure de couche (1) comprend en outre et/ou 2) la réalisation (104) de la deuxième structure de couche (5) comprend : 1) (c) la formation (105) d'une première couche de définition de cavité (7a) au-dessus du réseau d'éléments de transducteurs ultrasonores (4) et/ou 2) (a) la formation (106) d'une deuxième couche de définition de cavité (7b) au-dessus de la couche de plaque arrière (6) ; et la définition d'un réseau de cavités (8) dans la première couche de définition de cavité (7a) et/ou dans la deuxième couche de définition de cavité (7b) ;
3) la fixation (107), par collage de la première structure de couche (1) à la deuxième structure de couche (5), au réseau d'éléments transducteurs ultrasonores (4) étant placé entre la couche flexible frontale (3) et la couche de plaque arrière (6) ; et
4) le retrait (108) du premier substrat (2).

2. Procédé (100) selon la revendication 1, dans lequel les éléments transducteurs ultrasonores (4) comprennent des éléments piézoélectriques.

3. Procédé (100) selon la revendication 2, dans lequel un élément transducteur ultrasonore (4) dans le réseau est défini par un desdits éléments piézoélectriques et dans lequel chaque cavité dudit réseau de cavités (8) est associée à un élément piézoélectrique respectif et l'élément piézoélectrique est disposé entre la cavité (8) et la couche flexible frontale (3).

4. Procédé (100) selon une quelconque des revendications précédentes, dans lequel 2) la réalisation (104) de la deuxième structure de couche (5) comprend : la formation (104a) de la couche de plaque arrière (6) au-dessus d'un deuxième substrat (12).

5. Procédé (100) selon la revendication 4, dans lequel le procédé comprend en outre, après 3), le retrait (111) du deuxième substrat (12).

6. Procédé (100) selon une quelconque des revendications 4 ou 5, dans lequel 2) le dispositif (104) de la deuxième structure de couche (5) comprend en outre la formation (110) d'une couche de support acoustique (10) au-dessus du deuxième substrat (12) afin de réduire la transmission acoustique dirigée à l'opposé dudit objet pendant le fonctionnement.

7. Procédé (100) selon une quelconque des revendications précédentes, dans lequel 2) le dispositif (104) de la deuxième structure de couche (5) comprend la formation (109) d'un réseau de circuits de commande (9) au-dessus de la couche de plaque arrière (6).

8. Procédé (100) selon la revendication 7, dans lequel les éléments de transducteur ultrasonores (4) dans le réseau de ladite première structure de couche (1) sont connectés électriquement à des circuits de commande individuels (9) du réseau de ladite deuxième structure de couche (5), dans lequel le réseau de circuits de commande (9) est configuré pour actionner le réseau d'éléments de transducteur ultrasonores (4) dans ladite première structure de couche (1).

9. Procédé (100) selon la revendication 8, dans lequel le procédé comprend en outre 5) la formation (112) de connexions électriques (13) entre le réseau d'éléments de transducteur ultrasonores (4) et le réseau de circuits de commande (9).

10. Procédé (100) selon la revendication 9, dans lequel 5) la formation des connexions électriques (13) comprend la formation d'un premier via à travers la première structure de couche (1) vers un circuit de commande du réseau de circuits de commande (9) et la formation d'un deuxième via à travers la couche flexible frontale (3) vers un élément transducteur ultrasonore du réseau d'éléments transducteurs ultrasonores (4) et la formation d'une connexion métallique entre le circuit de commande (9) et l'élément transducteur ultrasonore (4) à travers le premier via, puis à travers le deuxième via.

11. Procédé (100) selon la revendication 9, dans lequel 5) la formation (112) des connexions électriques (13) consiste à former un via à travers la première structure de couche (1), comprenant un élément transducteur ultrasonore du réseau d'éléments transducteurs ultrasonores (4), vers un circuit de commande du réseau de circuits de commande (9) et la formation d'une connexion métallique entre le circuit de commande (9) et l'élément transducteur ultrasonore (4) à travers le via.

12. Procédé (100) selon une quelconque des revendications 7 à 11, comprenant en outre la disposition d'une structure de circuit intégré au-dessus ou au-dessous de la couche de plaque arrière (6) et la connexion de la structure de circuit intégré au réseau de circuits de commande (9).

13. Procédé (100) selon une quelconque des revendications précédentes, comprenant en outre 4), l'ajout (113) d'une couche supplémentaire (11) au-dessus de la couche flexible frontale (3).

14. Procédé (100) selon une quelconque des revendications précédentes, dans lequel 1) la formation (101) de la première structure de couche (1) et 2) la réalisation (104) de la deuxième structure de couche (5) sont traitées séparément.
